# EUROPÄISCHE PATENTSCHRIFT

(11) **EP 4 376 731 B1**
(45) Veröffentlichungstag und Bekanntmachung des Hinweises auf die Patenterteilung: **17.12.2025**
(21) Anmeldenummer: 22757863.0
(22) Anmeldetag: 25.07.2022
(51) Int. Cl.: A61B 17/29, A61B 34/00, A61B 17/00

(54) **LAGERUNGSANORDNUNG EINER TAUMELSCHEIBE IN EINEM LENKGETRIEBEBAUTEIL UND CHIRURGISCHES INSTRUMENT**
BEARING ASSEMBLY OF A SWASH PLATE IN A STEERING GEAR COMPONENT, AND SURGICAL INSTRUMENT
ENSEMBLE PALIER D'UN PLATEAU OSCILLANT DANS UN COMPOSANT DE MÉCANISME DE DIRECTION, ET INSTRUMENT CHIRURGICAL

(30) Priorität: 28.07.2021 DE 102021119529
(43) Veröffentlichungstag der Anmeldung: 05.06.2024
(73) Patentinhaber: Karl Storz SE & Co. KG, 78532 Tuttlingen (DE)
(72) Erfinder: SCHNEIDER, Janosz, 78532 Tuttlingen (DE); GRÜNER, Sven Axel, 78532 Tuttlingen (DE); LÄNGLE, Dominik, 78532 Tuttlingen (DE); STEFAN, Jochen, 78532 Tuttlingen (DE)
(86) Internationale Anmeldenummer: PCT/EP2022/070816
(87) Internationale Veröffentlichungsnummer: WO 2023/006676

(56) Entgegenhaltungen:
- WO-A2-2020/218920
- DE-A1- 102019 121 092

## Beschreibung

Die Erfindung betrifft eine Lagerungsanordnung einer Taumelscheibe in einem Lenkgetriebebauteil sowie ein chirurgisches Instrument, das eine solche Lagerungsanordnung aufweist.

Aus dem Stand der Technik sind chirurgische Instrumente bekannt, die manuell oder von einem Roboter geführt werden können, und die Werkzeuge aufweisen, deren Werkzeugspitze mittels mehrerer ineinandergreifender Schwenkglieder verschwenkt werden kann. Diese Schwenkglieder sind mit einer Vielzahl Lenkdrähte oder -seile verbunden, um eine feinfühlige Steuerung der Werkzeugspitze zu erreichen. Mit vielen dünnen Lenkdrähten gegenüber wenigen dickeren Lenkdrähten kann eine gleichmäßigere Kraftverteilung in alle Abwinkelungsrichtungen erzielt werden.

Aus US 5,454,827 B2 ist bekannt, solche Lenkdrähte mit einer proximalseitig in einer Betätigungseinheit angeordneten räumlich verstellbaren Scheibe zu koppeln, die über eine Stange mit einem manuell betätigbaren Steuerhebel verbunden ist, sodass eine Bewegung der räumlich verstellbaren Taumelscheibe eine entsprechende relative Bewegung der distalseitigen Schwenkglieder und somit ein Verschwenken der Werkzeugspitze verursacht.

Die Ausbildung des Antriebs für die Lenkdrähte mit der räumlich verstellbaren Taumelscheibe, an der die Lenkdrähte gelagert sind, hat den Vorteil, dass dies eine räumlich kompakte Bauweise ermöglicht und nur ein Bauteil bewegt werden muss, um alle Lenkdrähte ansprechen zu können.

US 10,105,128 B2 offenbart ein chirurgisches Instrument mit einer Mechanik, um die Bewegung einer Taumelscheibe in zwei Freiheitsgraden über Gelenkstangen mit proximalseitigen Antrieben zu steuern, allerdings ist die Taumelscheibe nicht im Instrument drehbar.

Ferner ist bekannt, in einem chirurgischen Instrument mit einem Lenkgetriebebauteil Antriebsstellwinkel auf eine Taumelscheibe zur räumlichen Ausrichtung in zwei Freiheitsgraden / Raumrichtungen zu übertragen, wobei das Lenkgetriebebauteil rotativ von der Taumelscheibe durch ein Kugellager entkoppelt ist. Durch eine kardanische Lagerung der Taumelscheibe auf einer Hauptwelle wird eine Rotationsbewegung des Instrumentenschafts auf die Taumelscheibe übertragen, sodass die räumliche Lage der Taumelscheibe durch eine Überlagerung der Bewegungen des Lenkgetriebebauteils und der Hauptwelle bestimmt wird.

Aus der DE 10 2019 121 092 A1 ist eine Lagerungsanordnung mit einer Taumelscheibe in einem Lenkgetriebebauteil eines chirurgischen Instruments, wobei die Taumelschiebe um eine Rotationsachse drehbar in einer Aufnahmeöffnung des Lenkgetriebebauteils gelagert ist, und wobei die Lagerungsanordnung eine Mehrzahl von Wälzkörpern aufweist, die Kugeln sind, bekannt.

Zudem ist aus der WO 2020/218920 A2 eine Lagerungsanordnung gemäß dem Oberbegriff des Anspruchs 1 bekannt.

Ausgehend von diesem Stand der Technik ist es Aufgabe der vorliegenden Erfindung, eine hinsichtlich des benötigten Bauraums verbesserte Lagerung einer Taumelscheibe in einem Lenkgetriebebauteil bereitzustellen, die eine Taumelscheibe in einem Lenkgetriebebauteil rotativ entkoppelt lagert.

Diese Aufgabe wird durch eine Lagerungsanordnung mit den Merkmalen des Anspruchs 1 gelöst.

Die weitere Aufgabe, ein chirurgisches Instrument bereitzustellen, das eine verbesserte Lagerungsanordnung der räumlich verstellbaren Taumelscheibe aufweist, wird durch das chirurgische Instrument mit den Merkmalen des unabhängigen Anspruchs 7 gelöst.

Weiterbildungen und bevorzugte Ausführungsformen der Lagerungsanordnung und des chirurgischen Instruments sind in den Unteransprüchen ausgeführt.

Gemäß einer ersten Ausführungsform der erfindungsgemäßen Lagerungsanordnung einer Taumelscheibe in einem Lenkgetriebebauteil, bei der die Taumelscheibe um eine Rotationsachse drehbar in einer Aufnahmeöffnung des Lenkgetriebebauteils gelagert ist, weist die Taumelscheibe eine Außenumfangsfläche auf, die eine umfängliche Innenlagerfläche bereitstellt. Das Lenkgetriebebauteil weist in der Aufnahmeöffnung eine Innenumfangsfläche auf, die eine umfängliche Außenlagerfläche bereitstellt, die in der Lagerungsanordnung axial korrespondierend zu der umfänglichen Innenlagerfläche der Taumelscheibe angeordnet ist. Ferner weist die Lagerungsanordnung eine Mehrzahl Wälzkörper auf, die Kugeln sind, wobei die Wälzkörper mit der umfänglichen Innenlagerfläche und der umfänglichen Außenlagerfläche ein Lager bilden, indem die Wälzkörper zwischen der umfänglichen Innenlagerfläche und der umfänglichen Außenlagerfläche umfänglich verteilt aufgenommen sind. Die umfängliche Innenlagerfläche und die umfängliche Außenlagerfläche weisen einen Querschnitt zur Ausbildung eines Vierpunkt-Lagers auf, um auch axiale Kräfte aufnehmen zu können. Eine Anzahl der Wälzkörper ist so ausgewählt, dass sie ein vollkugeliges Lager bereitstellt. Auf diese Weise entsteht ein Wälzlager, das in die Einheit aus Taumelscheibe und Lenkgetriebebauteil integriert ist. Da die Lageranordnung somit ohne Lagerinnen- und Lageraußenring auskommt, weil die Lagerlaufflächen in die Taumelscheibe und das Lenkgetriebebauteil integriert sind, wird weniger Bauraum benötigt und die Lageranordnung kann kompakt aufgebaut werden. Die Taumelscheibe selbst stellt somit den Innenring des Lagers und das Lenkgetriebebauteil den Außenring bereit.

Die "Flächen" bezeichnen hierbei Oberflächenabschnitte von Taumelscheibe und Lenkgetriebebauteil, wobei die jeweiligen "Flächen" nicht eben und stetig sein müssen, sondern auch strukturiert, also uneben und nicht stetig sein können. Unter der "Außenumfangsfläche" der Taumelscheibe ist deren Außenmantelfläche zwischen den Stirnseiten der Taumelscheibe zu verstehen, wobei die "Außenumfangsfläche" bzw. Außenmantelfläche der Taumelscheibe mit unterschiedlichen Abschnitten ausgeformt sein kann. Dabei ist zumindest ein Oberflächenabschnitt der "Außenumfangsfläche" als die "umfängliche Innenlagerfläche" ausgeformt, die für die Wälzkörper eine innere Laufbahn des integrierten Wälzlagers bildet. Entsprechendes gilt für die "Innenumfangsfläche" der Aufnahmeöffnung des Lenkgetriebebauteils mit der "umfänglichen Außenlagerfläche": Unter der "Innenumfangsfläche" ist die Innenmantelfläche des Lenkgetriebebauteils zwischen dessen Vorder- und Rückseite zu verstehen, wobei die "Innenumfangsfläche" bzw. Innenmantelfläche des Lenkgetriebebauteils die Aufnahmeöffnung definiert und mit unterschiedlichen Abschnitten ausgeformt sein kann. Dabei ist zumindest ein Oberflächenabschnitt der "Innenumfangsfläche" als die "umfängliche Außenlagerfläche" ausgeformt, die für die Wälzkörper eine äußere Laufbahn des integrierten Wälzlagers bildet. D. h., dass sowohl die "umfängliche Innenlagerfläche" als auch die "umfängliche Außenlagerfläche" beispielsweise auch jeweils aus zwei Flächenabschnitten, z. B. Kugelbahnabschnitten bestehen können, die spitz oder über einen Radius miteinander verbunden sein können.

Die Ausführung als Vierpunkt-Lager kann bevorzugt sein, da damit besonders gut im Betrieb auftretende axiale Kräfte in beide Richtungen abgefangen werden können. So können besonders auch die Kipp-Momente, die als zentraler Lastfall einer Taumelscheibe auftreten, durch ein Vierpunkt-Lager optimal abgefangen bzw. übertragen werden.

Vollkugelige Lager sind dabei bevorzugt, da sie höher belastbar sind als Ausführungen mit Käfig oder Trennkörpern, und gegenüber Ausführungen mit Lagerkäfigen einen geringeren Raumbedarf sowie gegenüber Ausführungen mit Trennkörpern einen geringeren Montageaufwand haben.

In einer weiteren bevorzugten Ausführungsform der erfindungsgemäßen Lagerungsanordnung wird eine Befüllvorrichtung zur Befüllung der zwischen der umfänglichen Innenlagerfläche und der umfänglichen Außenlagerfläche bereitgestellten Lagerlaufbahn dadurch vorgesehen, dass das Lenkgetriebebauteil eine Zugangsbohrung aufweist, die sich zu der umfänglichen Außenlagerfläche erstreckt und die mit einem Füllstopfen verschließbar ist. Selbstverständlich hat die Zugangsbohrung einen an die Dimensionen des Wälzkörpers angepassten Querschnitt, um das Befüllen zu ermöglichen. Unter einem Füllstopfen wird hierbei jedes Verschlussmittel verstanden, das geeignet ist eine solche Zugangsbohrung so zu verschließen, dass das Abrollen der in das Lager eingefüllten Wälzkörper entlang der Außenlagerfläche nicht beeinträchtigt wird.

Alternativ zu einer durch eine verschließbare Zugangsbohrung gebildeten Befüllvorrichtung kann die Taumelscheibe oder das Lenkgetriebebauteil zweiteilig ausgeführt sein, wobei eine Trennebene jeweils die umfängliche Innenlagerfläche oder die umfängliche Außenlagerfläche teilt. Dazu kann die Taumelscheibe an der Innenlagerfläche axial, und das Lenkgetriebebauteil an der Außenlagerfläche radial oder axial getrennt werden. Die Befüllung mit den Wälzkörpern erfolgt in einer Montageanordnung von Taumelscheibe und Lenkgetriebebauteil, wobei von dem zweiteiligen Bauteil nur ein erstes Bauteil montiert ist, und die Montage des zweiten Bauteils zur Bildung der Lagerungsanordnung erst nach erfolgter Befüllung erfolgt.

Eine weitere alternative Ausführungsform zur Befüllung des Lagers mit den Wälzkörpern kann vorsehen, dass die Taumelscheibe und das Lenkgetriebebauteil relativ zueinander in Richtung der Rotationsachse verschiebbar sind, sodass bei axialer Verschiebung ein Spalt im Lagerbereich entsteht, durch den die Wälzkörper eingefüllt werden können. Da hierzu eine Flanke bzw. ein Flächenabschnitt einer der Laufbahnen Außenlagerfläche oder Innenlagerfläche abweichend ausgeformt wird, ist diese Ausführungsform nur zur begrenzten Aufnahme von Lagerkräften geeignet. Ferner ist in dieser Ausführungsform die Anordnung einer Sicherungsvorrichtung sinnvoll, durch die der Montagespalt im Betrieb stets geschlossen bleibt.

Unter den Befüllvarianten ist die Ausführungsform mit verschließbarer Zugangsbohrung bevorzugt, da hierdurch konstruktive Nachteile vermieden werden können, wie sie durch eine zweiteilige Gestaltung der Taumelscheibe oder des Lenkgetriebebauteils oder durch eine ungünstige Wälzkörperführung bei verschiebbaren Taumelscheibe und Lenkgetriebebauteil entstehen können.

Die Taumelscheibe der erfindungsgemäßen Lagerungsanordnung ist in einer weiteren Ausführungsform auf einer Welle angeordnet, die die Rotationsachse definiert. Dazu weist die Welle einen Kugelabschnitt auf, an dem der Wellendurchmesser zur Bildung der Kugelkontur aufgeweitet ist, und die Taumelscheibe weist eine an den Kugelabschnitt zumindest teilweise angepasst konturierte Aufnahmeausnehmung an ihrer Innenseite auf, sodass die Taumelscheibe ohne Axialversatz um zwei Achsen senkrecht zur Rotationsachse beweglich auf der Welle gelagert ist. Um eine Rotation bzw. einen Drehwinkel der Welle auf die Taumelscheibe zu übertragen, liegen an dem Kugelabschnitt der Welle in ihrer Außenfläche zwei Führungsnuten vor, die sich diametral und in Längsrichtung der Welle erstrecken und in die zwei Stifte eingreifen, die diametral und radial nach innen weisend an der Taumelscheibe bzw. einer Innenseite der Taumelscheibe angeordnet sind.

Vorteilhaft ergibt sich so eine drehsteife Verbindung zwischen Welle und Taumelscheibe, die auch bei einem großen Winkelversatz (± 40 ° und mehr) eine Drehwinkelübertragung erlaubt, dabei aber trotzdem noch sehr kompakt aufgebaut, sowie einfach herzustellen und zu montieren ist.

Nach noch einer weiteren Ausführungsform er Lagerungsanordnung kann die Aufnahmeausnehmung in der Taumelscheibe korrespondierend zu dem Kugelabschnitt kugelförmig ausgebildet sein, wodurch die Aufnahmeausnehmung in der Art einer Gelenkpfanne beidseitig wirkt und die Kupplung damit axial vollständig festgelegt ist. Eine zweiteilige Ausführung der Taumelscheibe ermöglicht hierbei die Montage, indem die Taumelscheibenteile auf den Kugelabschnitt zur Fertigstellung der Taumelscheibe montiert werden.

Gemäß einer dazu alternativen Ausführungsform weist die Aufnahmeausnehmung in der Taumelscheibe einen kugelförmigen Abschnitt und einen zylindrischen oder sich aufweitenden Abschnitt auf, sodass die Taumelscheibe nur einseitig durch den kugelförmigen Abschnitt festgelegt ist und eine Montage auf dem Kugelabschnitt ermöglicht wird, indem der zylindrische oder sich aufweitende Abschnitt beim Aufschieben auf die Welle in Richtung des Kugelabschnitts weist.

Das erfindungsgemäße chirurgische Instrument, das eine Lagerungsanordnung einer Taumelscheibe in einem Lenkgetriebebauteil aufweist, sieht in einer ersten Ausführungsform vor, dass die Lagerungsanordnung eine erfindungsgemäße Lagerungsanordnung ist, da die erfindungsgemäße Lagerungsanordnung sich aufgrund der kompakten Bauweise besonders für den Verbau in einem chirurgischen Instrument eignet, das eine Hauptwelle und einen koaxial zu einer Längsachse der Hauptwelle verlaufenden hohlen Schaft aufweist. An dem proximalen Schaftende (also dem Betätiger näher liegenden Ende) ist eine Betätigungseinheit und an dem distalen Ende des Schafts ist eine Werkzeugspitze mit einem Werkzeug angeordnet, das über ein axial verschiebbar im Schaft gelagertes Betätigungselement betätigbar ist, das sich ferner durch eine längsaxiale Durchgangsbohrung der Hauptwelle erstreckt und proximalseitig mit der Betätigungseinheit in Wirkverbindung steht. Die erfindungsgemäße Lagerungsanordnung ist dabei zur räumlichen Ausrichtung der Taumelscheibe in Bezug auf die Hauptwelle im Zusammenwirken mit einem proximalseitigen Antrieb ausgebildet, sodass eine Bewegung des proximalseitigen Antriebs das Verschwenken der Werkzeugspitze verursacht.

Die Lagerungsanordnung weist mit dem Lenkgetriebebauteil eine Schnittstelle mit dem proximalseitigen Antrieb auf, um deren Stellwinkel auf die Taumelscheibe zur Steuerung der distalen Werkzeugspitze zu übertragen. Durch die räumliche Ausrichtung der Taumelscheibe in Bezug auf die Hauptwelle durch Bewegung des proximalseitigen Antriebs wird ein Verschwenken der Werkzeugspitze bewirkt, die über einen Gelenkmechanismus relativ zur Längsachse des Schaftes verschwenkbar ist. Der Gelenkmechanismus kann in einer Ausführungsform aus am distalen Ende des Schaftes angeordneten Schwenkgliedern bestehen, die über in Längsrichtung des Schaftes verlaufende Lenkdrähte mit dem proximalseitigen Antrieb verbunden sind, indem die Lenkdrähte an der Taumelscheibe gelagert sind, sodass eine Bewegung des proximalseitigen Antriebs eine entsprechende relative Bewegung der distalseitigen Schwenkglieder und somit das Verschwenken der Werkzeugspitze verursacht.

In einer bevorzugten Ausführungsform der Lagerungsanordnung des erfindungsgemäßen chirurgischen Instruments kann der proximalseitige Antrieb als motorisierter Antrieb mit zumindest zwei Antriebsrädern ausgebildet sein, z. B. angetriebenen Zahnrädern, zwischen denen die Taumelscheibe angeordnet ist.

In dieser Ausführungsform ist die Taumelscheibe durch die Lagerungsanordnung mit einem dritten Zahnrad gekoppelt, das mit den beiden Antriebsrädern in Eingriff steht, wobei das Lenkgetriebebauteil ein Lenkring ist, der drehfest mit dem dritten Zahnrad gekoppelt ist, wobei bevorzugt auf der Drehachse des dritten Zahnrads um 180 ° versetzt zum dritten Zahnrad ein viertes Zahnrad angeordnet ist, das mit den beiden angetriebenen Zahnrädern in Eingriff steht, sodass die gebildete Verzahnungskette zu einem Verzahnungsring geschlossen wird, der eine gleichmäßig umlaufende Kraftverteilung gewährleistet.

Weitere Ausführungsformen sowie einige der Vorteile, die mit diesen und weiteren Ausführungsformen verbunden sind, werden durch die nachfolgende ausführliche Beschreibung unter Bezug auf die begleitenden Figuren deutlich und besser verständlich. Gegenstände oder Teile derselben, die im Wesentlichen gleich oder ähnlich sind, können mit denselben Bezugszeichen versehen sein. Die Figuren sind lediglich eine schematische Darstellung einer Ausführungsform der Erfindung. In den Zeichnungen ist ein Ausführungsbeispiel der Erfindung dargestellt. Die Zeichnungen, die Beschreibung und die Ansprüche enthalten zahlreiche Merkmale in Kombination. Der Fachmann wird die Merkmale zweckmäßigerweise auch einzeln betrachten und zu sinnvollen weiteren Kombinationen zusammenfassen.

Dabei zeigen:
- Fig. 1: eine schematische perspektivische Seitenansicht eines chirurgischen Instruments,
- Fig. 2: eine perspektivische Detailansicht einer Taumelscheiben-Lagerungsanordnung aus dem Stand der Technik mit einem Kugellager zur Lagerung der Taumelscheibe in einem Lenkring des Antriebs für das chirurgische Instrument,
- Fig. 3: eine perspektivische Ansicht einer Lagerungsanordnung nach einer erfindungsgemäßen Ausführungsform,
- Fig. 4: eine Seitenschnittansicht der Lagerungsanordnung aus Fig. 3,
- Fig. 5: eine Explosionsansicht der Seitenschnittansicht aus Fig. 4 ohne Wälzkörper,
- Fig. 6: eine Detailschnittansicht D der Lagerungsanordnung aus Fig. 4,
- Fig. 7: eine Detailschnittansicht durch die als Vierpunkt-Lager ausgeführte Lagerungsanordnung aus Fig. 4,
- Fig. 8: eine Untenansicht auf die Lagerungsanordnung aus Fig. 3,
- Fig. 9: eine perspektivische Teilschnittansicht der auf einem Kugelabschnitt einer Hauptwelle angeordneten Taumelscheibe, die zur erfindungsgemäßen Lagerung in einem Lenkring eines Antriebs für ein chirurgisches Instrument ausgebildet ist,
- Fig. 10: eine Teilschnittdraufsicht der auf dem Kugelabschnitt der Hauptwelle angeordneten Taumelscheibe aus Fig. 8, und
- Fig. 11: eine perspektivische Ansicht einer Taumelscheibe zur Lagerung in einem Lenkring eines Antriebs für ein chirurgisches Instrument und zur Anordnung auf einem Kugelabschnitt der Hauptwelle.

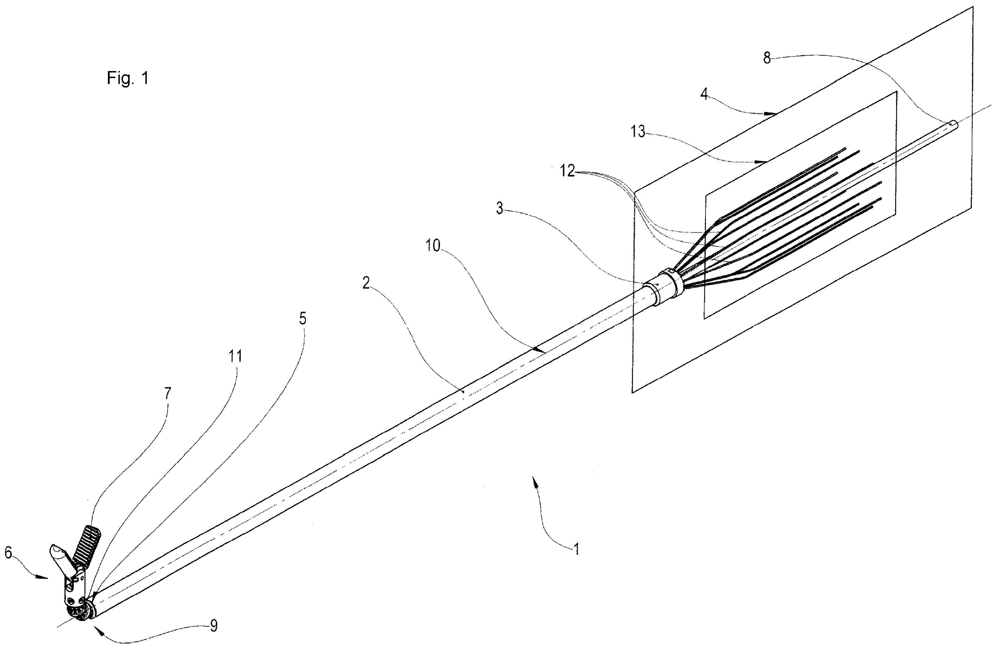

Fig. 1 zeigt schematisch ein chirurgisches Instrument 1 mit einem hohlen Schaft 2, einer am proximalen Ende 3 des Schaftes 2 angeordneten, nur schematisch dargestellten Betätigungseinheit 4 und einer am distalen Ende 5 des Schaftes 2 angeordneten Werkzeugspitze 6 mit einem Werkzeug 7, das über ein axial verschiebbar im Schaft 2 gelagertes Betätigungselement 8 betätigbar ist, das proximalseitig mit der Betätigungseinheit 4 in Wirkverbindung steht. Bei der Betätigungseinheit 4 kann es sich um eine manuell betätigbare Handhabe oder aber um eine für den robotischen Einsatz ausgelegte, also auch ohne manuelles Zutun betätigbare Baueinheit handeln. Bei dem Werkzeug 7 der Werkzeugspitze 6 kann es sich beispielsweise um ein mit Maulteilen versehenes Werkzeug, wie in Fig. 1 dargestellt, oder aber um ein Endoskop, einen Applikator oder dergleichen handeln. Die Werkzeugspitze 6 ist über einen Gelenkmechanismus 9 relativ zur Längsachse 10 des Schaftes 2 verschwenkbar, wobei der Gelenkmechanismus 9 aus am distalen Ende des Schaftes 5 angeordneten Schwenkgliedern 11 besteht, die über in Längsrichtung des Schaftes 2 verlaufende Lenkdrähte 12 so mit einem am proximalen Ende 3 des Schaftes 2 angeordneten Antrieb 13 verbunden sind, dass eine Bewegung des proximalseitigen Antriebs 13 eine entsprechende relative Bewegung der distalseitigen Schwenkglieder 11 und somit ein Verschwenken der Werkzeugspitze 6 verursacht. Auch wenn voranstehend und nachfolgend nur der Begriff Lenkdrähte 12 verwendet wird, können funktional auch Lenkseile verwendet werden, weshalb der verwendete Begriff Lenkdrähte 12 synonym auch als Lenkseil zu lesen und zu verstehen ist.

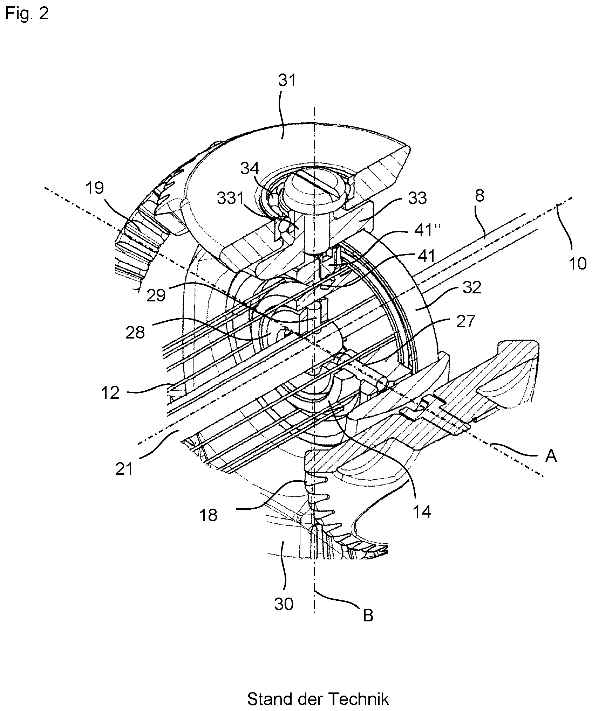

Das axialverschiebbar im Schaft 2 gelagerte Betätigungselement 8 zum Betätigen des beispielsweise aus zwei Maulteilen bestehenden Werkzeugs 7 ist bei den dargestellten Ausführungsformen als Zug- / Schubstange ausgebildet. Der Antrieb 13 für die Lenkdrähte 12 kann bei dem erfindungsgemäßen medizinischen Instrument 1 vorzugsweise als motorisierter Antrieb 13 ausgebildet sein, der eine räumlich verstellbare Taumelscheibe 14 aufweist, an der die Lenkdrähte 12 so gelagert sind, dass eine vorzugsweise über den motorisierten Antrieb 13 bewirkte Verlagerung der Taumelscheibe 14 über die Lenkdrähte 12 ein Verschwenken der Werkzeugspitze 6 bewirkt, wie aus dem Stand der Technik z. B. US 10,105,128 B2 oder der Taumelscheiben-Lagerungsanordnung aus Fig. 2 bekannt.

Durch die Verwendung eines motorisierten Antriebs 13 für die räumlich verstellbare Scheibe 14 ist es möglich, die Lenkdrähte 12 zum Verschwenken der distalseitigen Schwenkglieder 11 bzw. der Instrumentenspitze 6 exakt, feinfühlig in kleinsten Schritten und auch reproduzierbar anzusteuern. Darüber hinaus ist die Anzahl der zu verwendenden Lenkdrähte 12 für einen motorisierten Antrieb 13 unerheblich. Der motorisierte Antrieb 13 kann, wie aus dem Stand der Technik gemäß Fig. 2 bekannt, zwei Antriebseinheiten mit über Motoren angetriebenen Zahnräder 18 und 19, dort Kegelräder, aufweisen, zwischen denen die Taumelscheibe 14 angeordnet ist.

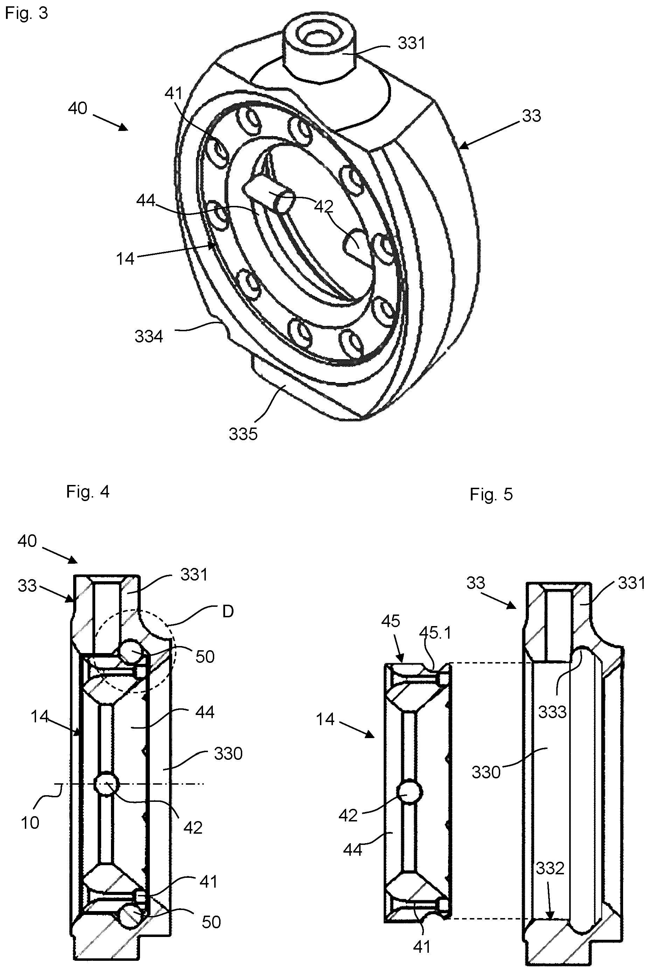

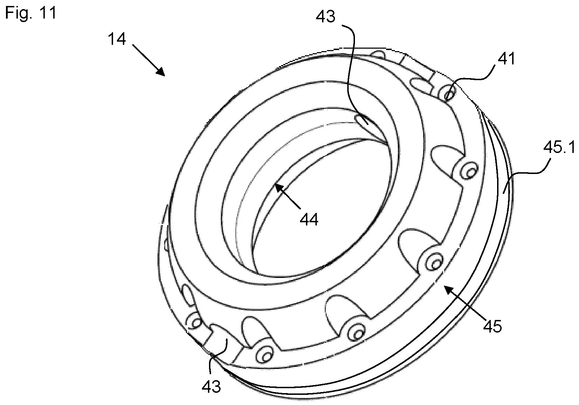

Bei dem chirurgischen Instrument 1 gemäß Fig. 1 (in Verbindung mit Fig. 2 - Stand der Technik - bzw. Fig. 3 bis 11 - erfindungsgemäße Lagerungsanordnung 40) ist im Schaft 2 eine sich koaxial zur Längsachse 10 des Schaftes 2 erstreckende hohle Hauptwelle 21 angeordnet, die um die Längsachse 10 des Schaftes 2 rotierbar ist und sich über das proximale Ende 3 des Schaftes 2 hinaus bis in den Bereich des motorisierten Antriebs 13 erstreckt. Innerhalb dieser hohlen Hauptwelle 21 ist axialverschiebbar das Betätigungselement 8 zur Betätigung des Instruments 7 gelagert.

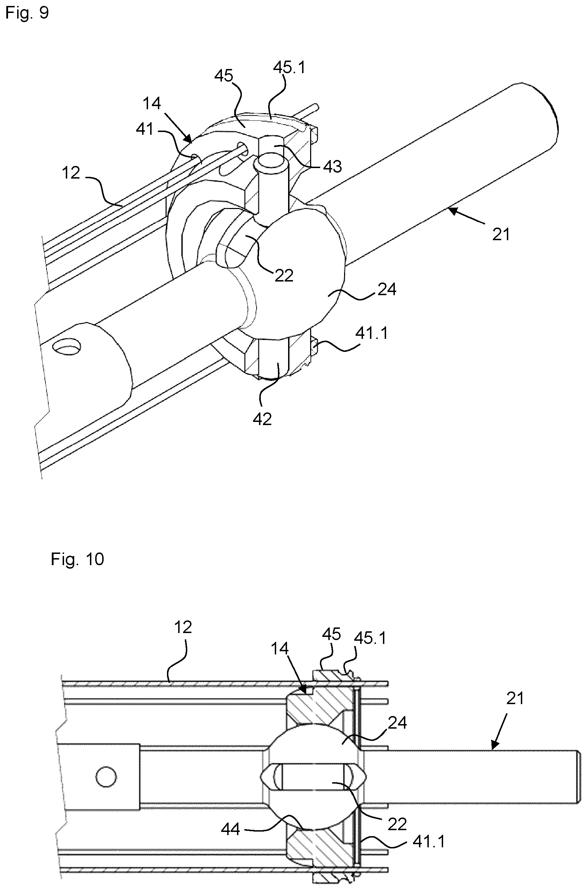

Die am proximalen Ende 3 des Schaftes 2 aus dem Schaft 2 austretenden Lenkdrähte 12 werden am distalen Ende der Hauptwelle 21 geführt und können über eine drehfest auf der Hauptwelle 21 angeordnete Fächerscheibe (nicht dargestellt) aufgefächert werden, wodurch der radiale Abstand der Lenkdrähte 12 von der Längsachse 10 des Schaftes 2 vergrößert wird. Die proximalseitig hinter einer solchen Fächerscheibe parallel zur Längsachse 10 des Schaftes 2 verlaufenden Lenkdrähte 12 erstrecken sich zur Taumelscheibe 14, an der die Lenkdrähte 12 festgelegt sind. Dazu weist die Taumelscheibe 14 eine axialparallele Durchgangsbohrung 41 für jeden Lenkdraht 12 auf, wobei die Lenkdrähte 12 innerhalb der Durchgangsbohrungen 41 über Madenschrauben 41.2 wie in Fig. 2 oder proximalseitig mit einer Klemmscheibe 41.1, wie in Fig. 9 und 10 dargestellt, fixierbar und kraftschlüssig mit der Taumelscheibe 14 verbindbar sind.

Die angetriebenen Zahnräder 18 und 19 sind mit einem dritten Zahnrad 30 gekoppelt, das mit den beiden angetriebenen Zahnrädern 18 und 19 in Eingriff steht und dessen Drehachse B die Mittelachse A der angetriebenen Zahnräder 18 und 19 sowie die Längsachse 10 des Schaftes 2 schneidet. Auch das dritte Zahnrad 30 ist vorzugsweise als Kegelrad ausgebildet. Durch die drei miteinander kämmenden Zahnräder 18, 19 und 30 wird jede Bewegung der beiden angetriebenen Zahnräder 18 und 19 direkt auf die mit dem dritten Zahnrad 30 gekoppelte Taumelscheibe 14 übertragen, was eine direkte Betätigung der Lenkdrähte 12 bewirkt. Um die durch die Zahnräder 18, 19 und 30 gebildete Verzahnungskette zu einem geschlossenen Verzahnungsring zu schließen, der eine gleichmäßig umlaufende Kraftverteilung gewährleistet, ist auf der Drehachse B des dritten Zahnrads 30 um 180 ° versetzt zum dritten Zahnrad 30 ein viertes Zahnrad 31 angeordnet, das mit den beiden angetriebenen Zahnrädern 18 und 19 in Eingriff steht, wobei auch das vierte Zahnrad 31 vorzugsweise als Kegelrad ausgebildet ist.

Die Taumelscheibe 14 ist in der bekannten Lagerungsanordnung gemäß Fig. 2 über einen (Kugel-)Lagerring 32 in dem drehfest mit dem dritten Zahnrad 30 gekoppelten Lenkring 33 gelagert, um eine Rotation der Taumelscheibe 14 um die Längsachse 10 des Schaftes 2 zu ermöglichen. Dabei ist der drehfest mit dem dritten Zahnrad 30 gekoppelte Lenkring 33 an einem Lagerstutzen 331 über einen Lagerring 34 frei drehbar gegenüber dem vierten Zahnrad 31, so dass eine Rotation des vierten Zahnrads 31 um seine Drehachse B keine Verdrehung des Lenkrings 33 und der Taumelscheibe 14 bewirkt. Die Verwendung eines Standard-Wälzlagers allerdings ist nur mit der Einschränkung eines erhöhten Bauraumbedarfs möglich, der einer Miniaturisierung eines damit ausgestatteten chirurgischen Instruments entgegensteht. Selbst ein Dünnringlager erfordert einen größeren Einbauraum, als zwischen Lenkring und Taumelscheibe bei der gewünschten Miniaturisierung verfügbar ist. Ein Gleitlager erfüllt zwar die Bauraumanforderungen, kann aber keine axialen Kräfte abfangen.

Die vorstehend in Bezug auf Fig. 2 beschriebenen Ausführungen des Antriebs 13 zur räumlichen Ausrichtung der Taumelscheibe 14 zur Steuerung der Werkzeugspitze mittels der Lenkdrähte 12 gelten auch für ein erfindungsgemäßes chirurgisches Instrument 1, das eine erfindungsgemäße Lagerungsanordnung 40 aufweist, wie sie in Fig. 3 bis 10 zu sehen ist.

Die erfindungsgemäße Lagerungsanordnung 40 weist anders als bei dem herkömmlichen Kugellager 32 keine Lagerringe auf, die eine Laufbahn für Wälzkörper 50 bereitstellen, um die Taumelscheibe 14 drehbar um Rotationsachse 10 in der Aufnahmeöffnung 330 des Lenkrings 33 zu lagern. Wie in Fig. 4 bis 6 zu erkennen ist, sind die Lagerlaufflächen bauraumsparend an der Taumelscheibe 14 und dem Lenkring 33 integriert, wobei die Taumelscheibe 14 an ihrer Außenumfangsfläche 45 eine umfängliche Innenlagerfläche 45.1 aufweist und damit die Funktion eines Lagerinnenrings übernimmt, während das Lenkgetriebebauteil 33 an der Innenumfangsfläche 332 der Aufnahmeöffnung 330 eine umfängliche Außenlagerfläche 333 aufweist, die die Funktion eines Lageraußenrings übernimmt. Die Wälzkörper 50, die bevorzugt Kugeln sind, damit das Lager nicht nur radiale, sondern auch axiale Belastungen aufnehmen kann, sind umfänglich verteilt in der durch die umfängliche Innenlagerfläche 45.1 und die umfängliche Außenlagerfläche 333 bereitgestellte Lagerlaufbahn aufgenommen.

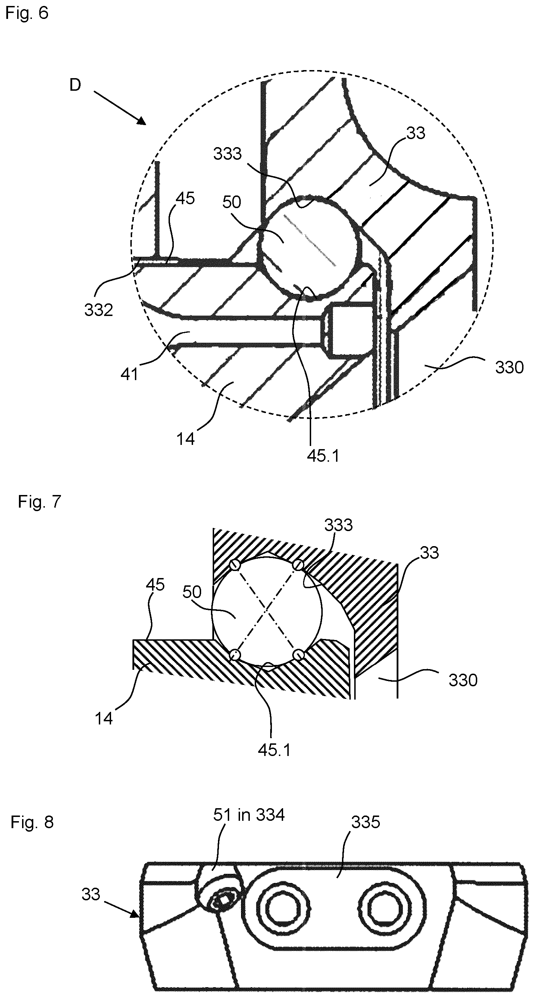

Mit Kugeln als Wälzkörper 50 weisen die umfängliche Innenlagerfläche 45.1 und die umfängliche Außenlagerfläche 333 einen kreisbogenförmigen Querschnitt zur Ausbildung eines nicht erfindungsgemäßen Rillenkugellagers auf, dessen Durchmesser an den Durchmesser der Kugeln 50 angepasst ist. Bevorzugt ist aber die Ausführung als Vierpunkt-Lager, wie in Fig. 7 skizziert, sodass axiale Belastungen in beide Richtungen aufgenommen werden können, wobei die umfängliche Innenlagerfläche 45.1 und die umfängliche Außenlagerfläche 333 jeweils einen Querschnitt aus zwei sich treffenden Kreisbögen aufweisen deren Durchmesser größer als der Durchmesser der Kugeln 50 ist, sodass die Kugeln nur an den eingezeichneten vier Punkten die Innenlagerfläche 45.1 und die Außenlagerfläche 333 kontaktieren.

Die Lageranordnung kann in einer nicht dargestellten nicht erfindungsgemäßen Ausführungsform einen Lagerkäfig oder Trennkörper zur gleichmäßigen Beabstandung der Wälzkörper aufweisen, wodurch eine Berührung benachbarter Wälzkörper miteinander vermieden wird, sodass eine Wärmeentwicklung infolge Reibung vermindert und eine gleichmäßige Lastverteilung über den Umfang unterstützt werden. Da solche Käfige oder Trennkörper allerdings zu einem erhöhten Bauraumbedarf führen können, ist eine vollkugelige Ausführung der Lageranordnung 40 bevorzugt, die zudem höher belastbar ist als eine Ausführung mit Käfig. Ein vollkugeliges Lager entsteht, indem die Summe der Durchmesser D aller n Wälzkörper (n*D) annähernd dem Umfang U ihrer Laufbahn entspricht, sodass gilt: U-(n*D) < D, es also nirgendwo große Spalten zwischen zwei benachbarten Wälzkörpern gibt.

In Fig. 8 ist eine durch einen Füllstopfen 51 verschlossene Zugangsbohrung 334 am Lenkring 33 zu sehen (in Fig. 3 angedeutet), die sich zu der umfänglichen Außenlagerfläche 333 erstreckt (nicht dargestellt). Die Zugangsbohrung 334 ermöglicht vor dem Verschluss mit einem Füllstopfen 51 die Befüllung der zwischen der Innenlagerfläche 45.1 der Taumelscheibe 14 und der Außenlagerfläche 333 des Lenkrings 33 gebildeten Lagerlaufbahn mit den Wälzkörpern 50. Ein Austausch kann entsprechend durch Entleeren von alten Wälzkörper durch die Zugangsbohrung 334 nach Entfernen des Füllstopfens 51 und Einfüllen neuer Wälzkörper erfolgen.

Alternativ zu der bevorzugten Befüllvorrichtung mittels Zugangsbohrung 334 kann eine erfindungsgemäße Lagerungsanordnung 40 mit Wälzkörpern befüllt werden, indem die Taumelscheibe 14 oder das Lenkgetriebebauteil 33 zweiteilig ausgeführt ist, wobei eine Trennebene jeweils die Innenlagerfläche 45.1 oder die Außenlagerfläche 333 teilt, oder indem die Taumelscheibe 14 und das Lenkgetriebebauteil 33 an der Außenumfangsfläche 45 und der Innenumfangsfläche 332 derart ausgebildet sind, dass sie relativ zueinander in Richtung der Rotationsachse 10 verschiebbar sind, sodass ein Spalt im Bereich der Innenlagerfläche 45.1 bzw. der Außenlagerfläche 333 entsteht, durch den das Lager befüllt werden kann.

Fig. 9 bis 11 verdeutlichen die Lagerung der Taumelscheibe 14 auf der Hauptwelle 21 eines chirurgischen Instruments 1 (vgl. Fig. 1). Die räumliche Ausrichtung der Taumelscheibe 14 zum Verschwenken der Werkzeugspitze 6 über die Lenkdrähte 12 wird durch eine Überlagerung der Rotationslage der Hauptwelle 21 und der Stellwinkel des proximalseitigen Antriebs (vgl. Fig. 2) bewirkt. Die Lagerungsanordnung ist konstruktiv einfach und kompakt aufgebaut sowie einfach zu montieren.

Die Hauptwelle 21 weist zur kugelgelenkigen Lagerung der Taumelscheibe 14 einen Kugelabschnitt 24 auf, an zwei sich in Längsrichtung der Welle 21 erstreckende und beidseitig bzw. diametral in den Kugelabschnitt 24 eingebrachte Führungsnuten 22 vorliegen. Die Taumelscheibe 14 weist eine an den Kugelabschnitt 24 zumindest teilweise angepasst konturierte Aufnahmeausnehmung 44 auf, von der sich zwei diametral und radial nach innen weisende Stifte 42 erstrecken, die in die Führungsnuten 22 am Kugelabschnitt 24 der Hauptwelle 21 eingreifen. Zur Montage der Stifte 42 kann die Taumelscheibe 14 zwei diametral radial verlaufende Durchtrittbohrungen 43 aufweisen, sodass die Stifte 42 von der Außenseite 45 der Taumelscheibe 14 durch die Durchtrittbohrung 43 eingeführt werden können, bis sie an der Innenseite der Taumelscheibe 14 austreten und in der gewünschten Länge radial nach innen vorstehen. Die Durchtrittbohrungen 43 sind axial von der umfänglichen Innenlagerfläche 45.1 an der Außenumfangsfläche 45 beabstandet, wie in Fig. 9 zu sehen ist. Alternativ zu Durchtrittbohrungen 43 können in nicht dargestellten Ausführungsformen die Stifte 42 in zwei diametral radial von der Innenseite aus verlaufende Sackbohrungen an der Taumelscheibe 14 befestigt werden, oder die Stifte 42 können an der Innenseite stoffschlüssig mit der Taumelscheibe 14 verbunden sein, z. B. einstückig gefertigt sein.

Die auf diese Weise kugelgelagerte Taumelscheibe 14 kann aus einer neutralen Position, in der die Taumelscheibe 14 in einer Ebene senkrecht zur Längs- bzw. Rotationsachse 10 liegt, um zwei senkrecht zur Rotationsachse 10 stehende Raumachsen (A, B, vgl. Fig. 2) verschwenkt werden. Durch den Eingriff der Stifte 42 in die Führungsnuten 22 kann zudem ein Drehwinkel der Welle 21 auf die Taumelscheibe 14 übertragen werden.

Das Verkippen bzw. Verdrehen der Taumelscheibe 14 um eine oder beide Drehachsen A, B relativ zur Längsachse 10 des Schaftes 2 bewirkt über die Lenkdrähte 12, dass distalseitig die Instrumentenspitze 6 in entsprechender Weise relativ zur Längsachse 10 des Schaftes 2 verschwenkt wird. Durch die Übertragung einer Rotationsbewegung der Hauptwelle 21 um die Längsachse 10 des Schaftes 2 auf die Taumelscheibe 14 kann die distalseitig mit der Hauptwelle 21 gekoppelte Werkzeugspitze 6 um die Längsachse 10 des Schaftes 2 rotiert werden.

In Fig. 10 ist zu sehen, dass die Aufnahmeausnehmung 44 im gezeigten Beispiel aus einem proximalseitigen kugelförmigen Abschnitt und einem distalseitigen zylindrischen Abschnitt besteht, der die Montage der Taumelscheibe 14 durch Aufschieben vom proximalseitigen Ende der Hauptwelle 21 erlaubt. Alternativ zu einem zylindrischen Abschnitt kann distalseitig ein sich aufweitender Abschnitt (nicht dargestellt) vorgesehen sein. Die Taumelscheibe 14 ist durch den proximalseitigen kugelförmigen Abschnitt nur einseitig auf dem Kugelabschnitt festgelegt, wird aber durch die Spannung der Lenkdrähte 12, die hier durch die Durchtrittöffnungen 41 hindurchgeführt sind, und mit einem proximalseitig angeordneten Klemmring 41.1 befestigt sind, gehalten, sodass die Taumelscheibe 14 in axialer Richtung festgelegt ist. In einer abgewandelten, ebenfalls nicht dargestellten Ausführungsform kann die Aufnahmeausnehmung 44 der Taumelscheibe 14 korrespondierend zu dem Kugelabschnitt 24 ohne zylindrischen oder aufweitenden Abschnitt nur mit einem kugelförmigen Abschnitt ausgebildet sein, sodass die kugelgelenkige Lagerung axial vollständig festgelegt ist. Diese Ausführungsform erfordert allerdings eine zumindest zweiteilige Ausführung der Taumelscheibe 14, um eine Montage auf dem Kugelabschnitt 24 zu ermöglichen.

In den Zeichnungen ist ein Ausführungsbeispiel der Erfindung dargestellt. Die Zeichnungen, die Beschreibung und die Ansprüche enthalten zahlreiche Merkmale in Kombination. Der Fachmann wird die Merkmale zweckmäßigerweise auch einzeln betrachten und zu sinnvollen weiteren Kombinationen zusammenfassen. Die vorliegende Erfindung stellt eine Lagerungsanordnung 40 einer Taumelscheibe 14 in einem Lenkgetriebebauteil 33 eines chirurgischen Instruments 1 bereit, wobei die Taumelscheibe 14 um eine Rotationsachse 10 drehbar in einer Aufnahmeöffnung 330 des Lenkgetriebebauteils 33 gelagert ist. Dabei weist die Taumelscheibe 14 eine Außenumfangsfläche 45 auf, die eine umfängliche Innenlagerfläche 45.1 bereitstellt, und das Lenkgetriebebauteil 33 weist in der Aufnahmeöffnung 330 eine Innenumfangsfläche 332 auf, die eine umfängliche Außenlagerfläche 333 bereitstellt. Ferner weist die Lagerungsanordnung 40 eine Mehrzahl von Wälzkörpern 50 auf, die umfänglich verteilt zwischen der umfänglichen Innenlagerfläche 45.1 der Taumelscheibe 14 und der umfänglichen Außenlagerfläche 333 des Lenkgetriebebauteils 33 aufgenommen ist, sodass durch die Taumelscheibe 14, das Lenkgetriebebauteil 33 und die Mehrzahl von Wälzkörpern 50 ein integriertes Wälzlager der Lagerungsanordnung 40 bereitgestellt wird. Ferner wird ein chirurgisches Instrument 1 offenbart, das die Lagerungsanordnung 40 aufweist.

### BEZUGSZEICHENLISTE

- 1: Medizinisches Instrument
- 2: Schaft
- 3, 5: Proximales, distales Ende (Schaft)
- 4: Betätigungseinheit
- 6, 7: Werkzeugspitze, Werkzeug
- 8: Betätigungselement
- 9: Gelenkmechanismus
- 10: Längsachse
- 11: Schwenkglied
- 12: Lenkdraht
- 13: Antrieb
- 14: Taumelscheibe
- 18, 19: Zahnrad (angetrieben)
- 21: Hauptwelle
- 22: Führungsnut
- 23: Durchgangsbohrung
- 24: Kugelabschnitt
- 27, 29: Lagerstift
- 28: Kreuzgelenkscheibe
- 30, 31: drittes, viertes Zahnrad
- 32, 34: Lagerring
- 33, 330, 331: Lenkring, Aufnahmeöffnung, Lagerstutzen
- 332, 333: Innenumfangsfläche, Außenlagerfläche
- 334, 335: Zugangsbohrung, Befestigungsfuß
- 40: Lagerungsanordnung
- 41, 41.1, 41.2: Durchgangsbohrung, Befestigungsscheibe, Madenschraube
- 42: Stift
- 43, 44: Aufnahmebohrung, Aufnahmeausnehmung
- 45, 45.1: Außenumfangsfläche, Innenlagerfläche
- 50: Wälzkörper
- 51: Füllstopfen

## Patentansprüche

1. Lagerungsanordnung (40) einer Taumelscheibe (14) in einem Lenkgetriebebauteil (33) eines chirurgischen Instruments (1), wobei die Taumelscheibe (14) um eine Rotationsachse (10) drehbar in einer Aufnahmeöffnung (330) des Lenkgetriebebauteils (33) gelagert ist, wobei die Lagerungsanordnung (40) eine Mehrzahl von Walzkörpern (50) aufweist, die Kugeln (50) sind,
wobei
die Taumelscheibe (14) eine Außenumfangsfläche (45) aufweist, die eine umfängliche Innenlagerfläche (45.1) bereitstellt, und
das Lenkgetriebebauteil (33) in der Aufnahmeöffnung (330) eine Innenumfangsfläche (332) aufweist, die eine umfängliche Außenlagerfläche (333) bereitstellt, und
die Mehrzahl von Wälzkörpern (50)umfänglich verteilt zwischen der umfänglichen Innenlagerfläche (45.1) der Taumelscheibe (14) und der umfänglichen Außenlagerfläche (333) des Lenkgetriebebauteils (33) aufgenommen ist, sodass durch die Taumelscheibe (14), das Lenkgetriebebauteil (33) und die Mehrzahl von Wälzkörpern (50) ein integriertes Wälzlager der Lagerungsanordnung (40) bereitgestellt wird, **dadurch gekennzeichnet, dass** die umfängliche Innenlagerfläche (45.1) und die umfängliche Außenlagerfläche (333) einen Querschnitt zur Ausbildung eines Vierpunkt-Lagers aufweisen, und eine Anzahl der Wälzkörper (50) so ausgewählt ist, dass die Mehrzahl von Wälzkörpern (50) ein vollkugeliges Lager bereitstellt.

2. Lagerungsanordnung (40) nach Anspruch 1,
**dadurch gekennzeichnet, dass**
- das Lenkgetriebebauteil (33) eine Zugangsbohrung (334) aufweist, die sich zu der umfänglichen Außenlagerfläche (333) erstreckt und die mit einem Füllstopfen (51) verschließbar ist, oder
- die Taumelscheibe (14) oder das Lenkgetriebebauteil (33) zweiteilig ausgeführt ist, wobei eine Trennebene jeweils die umfängliche Innenlagerfläche (45.1) oder die umfängliche Außenlagerfläche (333) teilt, oder
- die Taumelscheibe (14) und das Lenkgetriebebauteil (33) relativ zueinander in Richtung der Rotationsachse (10) verschiebbar sind.

3. Lagerungsanordnung (40) nach Anspruch 1 oder 2,
**dadurch gekennzeichnet, dass**
die Taumelscheibe (14) auf einer Welle (21), die die Rotationsachse (10) definiert, angeordnet ist, wobei die Welle (21) zur Lagerung der Taumelscheibe (14) einen Kugelabschnitt (24) aufweist, und die Taumelscheibe (14) eine an den Kugelabschnitt (24) zumindest teilweise angepasst konturierte Aufnahmeausnehmung (44) aufweist, wobei
- der Kugelabschnitt (24) zwei sich in Längsrichtung der Welle (21) erstreckende, diametral angeordnete Führungsnuten (22) aufweist und
- an der Taumelscheibe (14) zwei diametral und radial nach innen weisende Stifte (42) angeordnet sind,
wobei jeder Stift (42) in eine der Führungsnuten (22) eingreift, sodass ein Drehwinkel der Welle (21) auf die Taumelscheibe (14) übertragbar ist.

4. Lagerungsanordnung (40) nach Anspruch 3,
**dadurch gekennzeichnet, dass**
die Aufnahmeausnehmung (44) korrespondierend zu dem Kugelabschnitt (24) kugelförmig ausgebildet ist, wobei die Taumelscheibe (14) zweiteilig ausgeführt ist, oder
die Aufnahmeausnehmung (44) einen kugelförmigen Abschnitt (45) und einen zylindrischen oder sich aufweitenden Abschnitt (46) aufweist.

5. Chirurgisches Instrument (1) mit einer Lagerungsanordnung einer Taumelscheibe (14) in einem Lenkgetriebebauteil (33),
**dadurch gekennzeichnet, dass**
die Lagerungsanordnung eine Lagerungsanordnung (40) nach zumindest einem der Ansprüche 1 bis 4 ist.

6. Chirurgisches Instrument (1) nach Anspruch 5,
**dadurch gekennzeichnet, dass**
das chirurgische Instrument (1) mit einer koaxial zu einem hohlen Schaft (2) verlaufenden Hauptwelle (21) ausgebildet ist und eine am proximalen Ende (3) des Schaftes (2) angeordnete Betätigungseinheit (4) und eine am distalen Ende (5) des Schaftes (2) angeordnete Werkzeugspitze (6) mit einem Werkzeug (7) aufweist, das über ein axial verschiebbar im Schaft (2) gelagertes Betätigungselement (8) betätigbar ist, das sich durch eine längsaxiale Durchgangsbohrung (23) in der Hauptwelle (21) erstreckt und proximalseitig mit der Betätigungseinheit (4) in Wirkverbindung steht, wobei die Werkzeugspitze (6) über einen Gelenkmechanismus (9) relativ zur Längsachse (10) des Schaftes (2) verschwenkbar ist, und der Gelenkmechanismus (9) mit einem proximalseitigen Antrieb (13) in Wirkverbindung steht, der die Taumelscheibe (14) aufweist.

7. Chirurgisches Instrument (1) nach Anspruch 5 oder 6,
**dadurch gekennzeichnet, dass**
der Gelenkmechanismus (9) aus am distalen Ende (5) des Schaftes (2) angeordneten Schwenkgliedern (11) besteht, die über in Längsrichtung des Schaftes (2) verlaufende Lenkdrähte (12) so mit dem proximalseitigen Antrieb (13) verbunden sind, dass eine Bewegung des proximalseitigen Antriebs (13) eine entsprechende relative Bewegung der distalseitigen Schwenkglieder (11) und somit ein Verschwenken der Werkzeugspitze (6) verursacht, wobei die Lenkdrähte (12) an der Taumelscheibe (14) gelagert sind.

8. Chirurgisches Instrument (1) nach zumindest einem der Ansprüche 5 bis 7,
**dadurch gekennzeichnet, dass**
der proximalseitige Antrieb (13) als motorisierter Antrieb (13) mit zumindest zwei Antriebsrädern (18, 19) ausgebildet ist, zwischen denen die Taumelscheibe (14) angeordnet ist.

9. Chirurgisches Instrument (1) nach Anspruch 8,
**dadurch gekennzeichnet, dass**
die Taumelscheibe (14) durch die Lagerungsanordnung (40) mit einem dritten Zahnrad (30) gekoppelt ist, das mit den beiden Antriebsrädern (18, 19) in Eingriff steht, wobei das Lenkgetriebebauteil (33) ein Lenkring (33) ist, der drehfest mit dem dritten Zahnrad (25) gekoppelt ist,
und wobei bevorzugt auf der Drehachse des dritten Zahnrads (30) um 180 ° versetzt zum dritten Zahnrad (30) ein viertes Zahnrad (31) angeordnet ist, das mit den beiden angetriebenen Zahnrädern (18, 19) in Eingriff steht.

## Claims

1. Bearing assembly (40) of a swash plate (14) in a steering mechanism component (33) of a surgical instrument (1), wherein the swash plate (14) is mounted in a receiving opening (330) of the steering mechanism component (33) so as to be rotatable about a rotational axis (10), wherein the bearing assembly (40) has a plurality of rolling elements (50) which are spheres (50),
wherein
the swash plate (14) has an outer circumferential surface (45) which provides a circumferential inner bearing surface (45.1), and
the steering mechanism component (33), in the receiving opening (330),
has an inner circumferential surface (332) which provides a circumferential outer bearing surface (333), and
the plurality of rolling elements (50) are accommodated so as to be circumferentially distributed between the circumferential inner bearing surface (45.1) of the swash plate (14) and the circumferential outer bearing surface (333) of the steering mechanism component (33), such that an integrated roller bearing of the bearing assembly (40) is provided by the swash plate (14), the steering mechanism component (33) and the plurality of rolling elements (50), **characterized in that** the circumferential inner bearing surface (45.1) and the circumferential outer bearing surface (333) have a cross section for forming a four-point bearing, and a number of the rolling elements (50) is selected such that the plurality of rolling elements (50) provides a full-complement ball bearing.

2. Bearing assembly (40) according to claim 1,
**characterized in that**
- the steering mechanism component (33) has an access bore (334) which extends to the circumferential outer bearing surface (333) and which can be closed with a filling plug (51), or
- the swash plate (14) or the steering mechanism component (33) is designed in two parts, wherein a parting plane divides the circumferential inner bearing surface (45.1) or the circumferential outer bearing surface (333) in each case, or
- the swash plate (14) and the steering mechanism component (33) are movable relative to one another in the direction of the rotational axis (10).

3. Bearing assembly (40) according to claim 1 or claim 2,
**characterized in that**
the swash plate (14) is arranged on a shaft (21) which defines the rotational axis (10), wherein the shaft (21) has a spherical portion (24) for supporting the swash plate (14), and the swash plate (14) has a receiving recess (44) which is contoured so as to be at least partially adapted to the spherical portion (24), wherein
- the spherical portion (24) has two diametrically arranged guide grooves (22) extending in the longitudinal direction of the shaft (21) and
- two diametrically and radially inwardly pointing pins (42) are arranged on the swash plate (14),
wherein each pin (42) engages in one of the guide grooves (22), such that an angle of rotation of the shaft (21) can be transferred to the swash plate (14).

4. Bearing assembly (40) according to claim 3,
**characterized in that**
the receiving recess (44) is spherical in shape, corresponding to the spherical portion (24), wherein the swash plate (14) is designed in two parts,
or
the receiving recess (44) has a spherical portion (45) and a cylindrical or widening portion (46).

5. Surgical instrument (1) comprising a bearing assembly of a swash plate (14) in a steering mechanism component (33),
**characterized in that**
the bearing assembly is a bearing assembly (40) according to at least one of claims 1 to 4.

6. Surgical instrument (1) according to claim 5,
**characterized in that**
the surgical instrument (1) is formed with a main shaft (21) running coaxially to a hollow sheath (2) and has an actuating unit (4) arranged at the proximal end (3) of the sheath (2) and a tool tip which is (6) arranged at the distal end (5) of the sheath (2) and has a tool (7) which can be actuated by an actuating element (8) mounted in an axially movable manner in the sheath (2), which element extends through a longitudinally axial through-bore (23) in the main shaft (21) and is operatively connected to the actuating unit (4) on the proximal side, wherein the tool tip (6) is pivotable relative to the longitudinal axis (10) of the sheath (2) via a joint mechanism (9), and the joint mechanism (9) is operatively connected to a proximal-side drive (13) which has the swash plate (14).

7. Surgical instrument (1) according to claim 5 or claim 6,
**characterized in that**
the joint mechanism (9) consists of pivoting members (11) arranged at the distal end (5) of the sheath (2), which are connected to the proximal-side drive (13) via steering wires (12) running in the longitudinal direction of the sheath (2) in such a way that a movement of the proximal-side drive (13) causes a corresponding relative movement of the distal-side pivoting members (11) and thus a pivoting of the tool tip (6), wherein the steering wires (12) are mounted on the swash plate (14).

8. Surgical instrument (1) according to at least one of claims 5 to 7,
**characterized in that**
the proximal-side drive (13) is designed as a motorized drive (13) having at least two drive wheels (18, 19) between which the swash plate (14) is arranged.

9. Surgical instrument (1) according to claim 8,
**characterized in that**
the swash plate (14) is coupled by the bearing assembly (40) to a third gear (30) which is in engagement with the two drive wheels (18, 19), wherein the steering mechanism component (33) is a steering ring (33) which is coupled to the third gear (25) for conjoint rotation,
and wherein a fourth gear (31) is preferably arranged on the rotational axis of the third gear (30), offset by 180° from the third gear (30), which fourth gear is in engagement with the two driven gears (18, 19).

## Revendications

1. Système formant palier (40) d'un plateau oscillant (14) dans un composant formant mécanisme de direction (33) d'un instrument chirurgical (1), dans lequel le plateau oscillant (14) est monté de manière à pouvoir tourner autour d'un axe de rotation (10) dans une ouverture de réception (330) du composant formant mécanisme de direction (33), dans lequel le système formant palier (40) présente une pluralité de corps de roulement (50), lesquels sont des billes (50),
dans lequel
le plateau oscillant (14) présente une surface circonférentielle extérieure (45) fournissant une surface de palier intérieure circonférentielle (45.1), et
le composant formant mécanisme de direction (33) présente dans l'ouverture de réception (330) une
surface circonférentielle intérieure (332) fournissant une surface de palier extérieure circonférentielle (333), et
la pluralité de corps de roulement (50) est reçue de manière répartie de manière circonférentielle entre la surface de palier intérieure circonférentielle (45.1) du plateau oscillant (14) et la surface de palier extérieure circonférentielle (333) du composant formant mécanisme de direction (33), de sorte qu'un palier à roulement intégré du système formant palier (40) est fourni par le plateau oscillant (14), le composant formant mécanisme de direction (33) et la pluralité de corps de roulement (50), **caractérisé en ce que** la surface de palier intérieure circonférentielle (45.1) et la surface de palier extérieure circonférentielle (333) présentent une section transversale pour la formation d'un palier à quatre points, et un certain nombre de corps de roulement (50) est choisi de sorte que la pluralité de corps de roulement (50) fournit un palier entièrement sphérique.

2. Système formant palier (40) selon la revendication 1,
**caractérisé en ce que**
- le composant formant mécanisme de direction (33) présente un trou d'accès (334) s'étendant jusqu'à la surface de palier extérieure circonférentielle (333) et pouvant être fermé par un bouchon de remplissage (51), ou
- le plateau oscillant (14) ou le composant formant mécanisme de direction (33) est conçu en deux parties, dans lequel un plan de séparation divise respectivement la surface de palier intérieure circonférentielle (45.1) ou la surface de palier extérieure circonférentielle (333), ou
- le plateau oscillant (14) et le composant formant mécanisme de direction (33) peuvent être coulissés l'un par rapport à l'autre en direction de l'axe de rotation (10).

3. Système formant palier (40) selon la revendication 1 ou 2,
**caractérisé en ce que**
le plateau oscillant (14) est disposé sur un arbre (21) définissant l'axe de rotation (10), dans lequel l'arbre (21) présente une section sphérique (24) pour le montage du plateau oscillant (14), et le plateau oscillant (14) présente un évidement de réception (44) profilé et au moins partiellement adapté à la section sphérique (24), dans lequel
- la section sphérique (24) présente deux rainures de guidage (22) disposées de manière diamétralement opposée, s'étendant dans la direction longitudinale de l'arbre (21), et
- deux broches (42) orientées diamétralement et radialement vers l'intérieur sont disposées sur le plateau oscillant (14),
dans lequel chaque broche (42) vient en prise dans l'une des rainures de guidage (22) de sorte qu'un
angle de rotation de l'arbre (21) peut être transmis au plateau oscillant (14).

4. Système formant palier (40) selon la revendication 3,
**caractérisé en ce que**
l'évidement de réception (44) est réalisé en forme de sphère correspondant à la section sphérique (24), dans lequel le plateau oscillant (14) est conçu en deux parties,
ou
l'évidement de réception (44) présente une section sphérique (45) et une section cylindrique ou évasée (46).

5. Instrument chirurgical (1) comportant un système formant palier d'un plateau oscillant (14) dans un composant formant mécanisme de direction (33),
**caractérisé en ce que**
le système formant palier est un système formant palier (40) selon au moins l'une des revendications 1 à 4.

6. Instrument chirurgical (1) selon la revendication 5,
**caractérisé en ce que**
l'instrument chirurgical (1) est réalisé avec un arbre principal (21) s'étendant coaxialement à une tige (2) creuse et présente une unité d'actionnement (4) disposée à l'extrémité proximale (3) de la tige (2) et une pointe d'outil (6) disposée à l'extrémité distale (5) de la tige (2) et comportant un outil (7) pouvant être actionné par l'intermédiaire d'un élément d'actionnement (8) monté de manière à pouvoir être axialement coulissé dans la tige (2),
lequel élément d'actionnement s'étend à travers un trou de passage (23) axial longitudinal dans l'arbre principal (21) et est en liaison fonctionnelle côté proximal avec l'unité d'actionnement (4), dans lequel la pointe d'outil (6) peut pivoter par rapport à l'axe longitudinal (10) de la tige (2) par l'intermédiaire d'un mécanisme d'articulation (9), et le mécanisme d'articulation (9) est en liaison fonctionnelle avec un entraînement (13) côté proximal présentant le plateau oscillant (14).

7. Instrument chirurgical (1) selon la revendication 5 ou 6,
**caractérisé en ce que**
le mécanisme d'articulation (9) est constitué d'éléments pivotants (11) disposés à l'extrémité distale (5) de la tige (2) et reliés à l'entraînement (13) côté proximal par l'intermédiaire de fils de direction (12) s'étendant dans la direction longitudinale de la tige (2) de sorte qu'un déplacement de l'entraînement (13) côté proximal provoque un déplacement relatif correspondant des éléments pivotants (11) côté distal et ainsi un pivotement de la pointe d'outil (6), dans lequel les fils de direction (12) sont montés sur le plateau oscillant (14).

8. Instrument chirurgical (1) selon au moins l'une des revendications 5 à 7,
**caractérisé en ce que**
l'entraînement (13) côté proximal est réalisé sous forme d'entraînement motorisé (13) comportant au moins deux roues d'entraînement (18, 19) entre lesquelles est disposé le plateau oscillant (14).

9. Instrument chirurgical (1) selon la revendication 8,
**caractérisé en ce que**
le plateau oscillant (14) est accouplé à une troisième roue dentée (30) par le système formant palier (40), laquelle est en prise avec les deux roues d'entraînement (18, 19), dans lequel le composant formant mécanisme de direction (33) est une bague de direction (33) accouplée de manière solidaire en rotation à la troisième roue dentée (25),
et dans lequel, de préférence sur l'axe de rotation de la troisième roue dentée (30), est disposée, de manière décalée de 180° par rapport à la troisième roue dentée (30), une quatrième roue dentée (31) en prise avec les deux roues dentées (18, 19) entraînées.
